**Europäisches Patentamt**

**European Patent Office**

**Office européen des brevets**

(19)

(11) Veröffentlichungsnummer: **0 321 648**

**A1**

(12) # EUROPÄISCHE PATENTANMELDUNG

(21) Anmeldenummer: 88112684.1

(22) Anmeldetag: 04.08.88

(51) Int. Cl.4: **A61M 25/00**

(30) Priorität: 21.11.87 DE 3739556

(43) Veröffentlichungstag der Anmeldung:
28.06.89 Patentblatt 89/26

(84) Benannte Vertragsstaaten:
**AT BE CH DE ES FR LI LU**

(71) Anmelder: **B. Braun Melsungen AG**
**Carl-Braun Strasse**
**D-3508 Melsungen(DE)**

(72) Erfinder: **Haindl, Hans, Dr.**
**Forstgarten 22**
**D-3508 Melsungen(DE)**
Erfinder: **Koch, Heinrich, Dr. Dipl.-Phys.**
**Haarbreite 41**
**D-3436 Hess. Lichtenau(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Katheter.**

(57) Der als Spülkatheter oder Schlürfsonde verwendbare Katheter (10) besteht aus einem flexiblen Katheterschlauch (11) mit geschlossener Spitze (12), dessen Lumen durch eine gewölbte Trennwand (22) in ein Hauptlumen (20) und ein kleineres Nebenlumen (21) unterteilt ist. Das Hauptlumen (20) steht über Öffnungen (25) in der Wand (26) des Katheterschlauches (11) mit der äußeren Umgebung in Verbindung und ein Schlitz (30) in der Trennwand (22) verbindet das Hauptlumen (20) mit dem Nebenlumen (21). Der Schlitz (30) ist als Ventil wirksam, das unter dem Druck eines Spülfluids aus dem Nebenlumen (21) öffnet, dieses jedoch gegen das Eindringen von Körperfluid aus dem Hauptlumen (20) in das Nebenlumen (21) absperrt. Auf diese Weise werden Verkrustungen des Nebenlumens (21) unterbunden und dieses kann nicht verstopft werden.

FIG. 1

FIG. 5

EP 0 321 648 A1

# Katheter

Die Erfindung bezieht sich auf einen Katheter, bestehend aus einem flexiblen Katheterschlauch mit geschlossener Spitze, dessen Lumen durch axial verlaufende Trennwände in parallele Haupt- und Nebenlumina unterteilt ist, wobei dem Hauptlumen Öffnungen in der Wand des Katheterschlauches zugeordnet sind und mindestens ein Durchlaß in dem spitzennahen Bereich jeder Trennwand das Hauptlumen mit einem Nebenlumen verbindet.

Derartige Katheter dienen beispielsweise als Drainagen und als Schlürfsonden für die Gastrektomie, wobei im ersten Falle das Nebenlumen von Spülflüssigkeit und im zweiten Falle von Luft durchströmt ist. Zahlreiche Körperflüssigkeiten haben die Eigenschaft, das Katheterlumen, durch das sie fließen, durch Verkrustung oder Ablagerung zu verschließen. Dies soll durch den Einsatz von Spülflüssigkeit, ggf. unter Hinzugabe von Medikamenten, die zur Auflösung von Krusten geeignet sind, verhindert werden, indem die Spülflüssigkeit von der Spitze des Katheters her aus dem kleineren Nebenlumen in das die Körperflüssigkeit führende Hauptlumen eingespült wird. Da der Durchlaß in der Trennwand zwischen Haupt- und Nebenlumen bei bekannten derartigen Kathetern und Drainagen aus offenen Durchbrüchen besteht, tritt das Problem auf, daß auch das kleine Nebenlumen mit Körperfluid in Berührung kommt und aufgrund seines kleineren Durchmessers in bezug auf das Hauptlumen noch schneller verkrustet als dieses. Der Strom der Spülflüssigkeit wird gedrosselt oder sogar vollständig abgesperrt und das Hauptlumen unterliegt ohne Spüleffekt verstärkter Verkrustung. Diese Problematik ergibt sich auch bei Anwendung des Katheters als Magensonde für gastrektomierte Patienten, wenn Luft durch den offenen Trennwanddurchlaß in das Hauptlumen einströmt, um zur Verhinderung des Ansaugens der Magenschleimhaut an die Hauptlumen-Öffnungen innerhalb des Hauptlumens einen Druckausgleich zu erzeugen.

Der Erfindung liegt die Aufgabe zugrunde, einen Katheter der eingangs erwähnten Art so zu verbessern, daß der Trennwanddurchlaß zum Nebenlumen und das Nebenlumen selbst gegen Verschluß durch Verkrustung geschützt werden.

Diese Aufgabe wird erfindungsgemäß dadurch gelöst, daß der Durchlaß in der Trennwand mit gewölbtem Querschnitt als Schlitz ausgebildet ist, dessen Lippen dicht aneinander liegen.

Auf diese Weise wird der Durchlaß in der Trennwand zwischen Haupt- und Nebenlumen mit einem Ventil versehen, das ein Einströmen von Luft oder Spülflüssigkeit aus dem Nebenlumen in das Hauptlumen ermöglicht, jedoch eine Fluid-Strömung in entgegengesetzter Richtung verhindert. Da die durch das Hauptlumen des Katheters fließende Körperflüssigkeit durch den geschlossenen Trennwanddurchlaß nicht in das Nebenlumen eintreten kann, wird dieses von der Körperflüssigkeit freigehalten und kann nicht durch Verkrustung oder Ablagerungen verschlossen werden. Spülflüssigkeit strömt ungehindert durch das Nebenlumen zur Spitze des Katheterschlauches und die sich öffnende Ventilanordnung in das Hauptlumen hinein und verhindert zuverlässig Verengungen seines lichten Durchflußquerschnitts durch Verkrustung oder Ablagerungen. Die Ventilanordnung sorgt auch bei Verwendung des Katheters als Schlürfsonde für eine ungehinderte Luft-Zuführung aus dem Nebenlumen in das Hauptlumen und verhindert das Eintreten von Körperfluid in das Nebenlumen, so daß der Luftzutrittskanal nicht verkrustet und eine einwandfreie Funktion der Schlürfsonde gewährleistet ist.

Die Unteransprüche kennzeichnen vorteilhafte Ausgestaltungen der Erfindung nach Anspruch 1.

Das Merkmal des Anspruches 2 hat den Vorteil, daß eine ausgezeichnete Abdichtwirkung der Schlitzlippen gegen das Einströmen von Körperflüssigkeit aus dem Hauptlumen in das Nebenlumen erreicht wird. Die Öffnung des Schlitzes zum Austritt von Flüssigkeit oder Luft aus dem Nebenlumen in das Hauptlumen erfolgt leichtgängig, so daß der Staudruck an der verschlossenen Spitze des Katheterschlauches zur Öffnung genügen kann.

Die Merkmale der Ansprüche 3 und 4 beziehen sich auf zweckmäßige Gestaltungen der Ventilanordnung, die dafür sorgen, daß eine einwandfreie Funktion des Schlitzverschlusses gewährleistet ist. Ggf. können mehrere Schlitze hintereinander angeordnet sein. Die Trennwand kann teilkreisförmigen oder ovalen Querschnitt haben, ohne daß die Funktion des Schlitzes als Einwegeventil beeinträchtigt wird.

Anspruch 4 bezieht sich auf eine alternative Anordnung des Schlitzes.

In der Zeichnung sind Ausführungbeispiele der Erfindung schematisch dargestellt. Es zeigen:

Fig. 1 eine Draufsicht auf einen als Schlürfsonde verwendbaren Katheter, teilweise im Längsschnitt,

Fig. 2 eine vergrößerte längsgeschnittene Ansicht der Spitze des Katheters,

Fig. 3 die um 90° gedrehte Spitze des Katheters nach Figur 2,

Fig. 4 einen Querschnitt durch die Spitze des Katheters bei geschlossenem Ventil und

Fig. 5 einen Querschnitt des Katheters bei geöffnetem Ventil,

Fig. 6 eine Draufsicht auf einen als Drainage mit Spüllumen verwendbaren Katheter, teilweise im Längsschnitt.

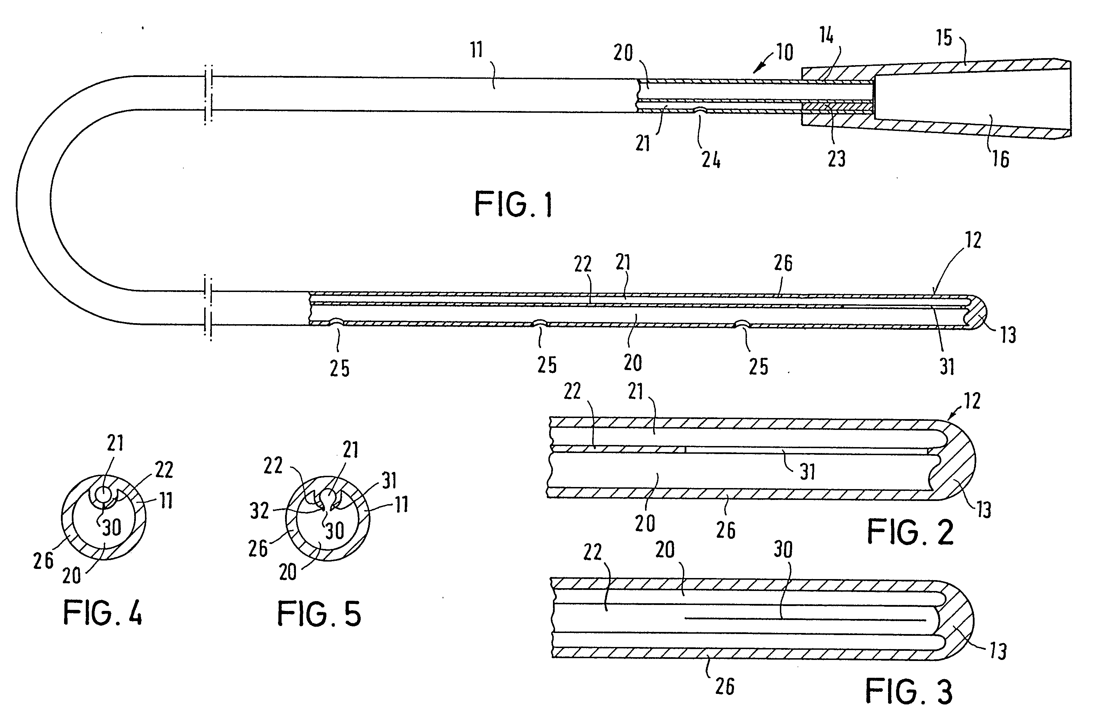

Der Katheter 10 der in Figur 1 dargestellten Schlürfsonde für gastrektomierte Patienten besteht im wesentlichen aus einem flexiblen Katheterschlauch 11, dessen Spitze 12 durch Verschmelzung bei 13 verschlossen ist und an dessen patientenfernem Ende 14 ein Ansatzstück 15 befestigt ist. Das Ansatzstück 15 ist mit einem Innenkonus 16 für eine Steckverbindung mit einem Außenkonus einer Sammelvorrichtung für Körperfluid versehen. Das Lumen des Katheterschlauches 11 ist in ein Hauptlumen 20 größeren Querschnitts und ein Nebenlumen 21 kleineren Querschnitts unterteilt. Diese Unterteilung erfolgt mit Hilfe einer im Querschnitt teilkreisförmigen Trennwand 22, die von einem Ende des Katheterschlauches 11 zum anderen kontinuierlich parallel zur zylindrischen Wand 26 des Katheterschlauches 11 verläuft. Während der Durchlaß des Hauptlumens 20 gegen den Innenkonus 16 des Ansatzstückes 15 offen ist, ist das Nebenlumen 21 durch einen Einsatz 23 mit zylindrischem Querschnitt an diesem Ende verschlossen. Ein Loch 24 in der Wand 26 des Katheterschlauches 11 in der Nähe des Ansatzstückes 15 dient zum Einlaß von Luft in das Nebenlumen 21. Weitere Öffnungen 25 in der Wand 26 an dem intrakorporalen Ende des Katheterschlauches 11 stellen eine Verbindung des Hauptlumens 20 mit der Umgebung, z.B. dem Magenhohlraum, her, aus dem Körperflüssigkeit abgezogen werden soll.

Die gebogene Trennwand 22 ist in dem an die Verschmelzung 13 des Katheterschlauches 11 unmittelbar anschließenden Bereich mit einem geraden, längsverlaufenden Schlitz 30 versehen, dessen Lippen 31, 32 im Schließzustand glattflächig dicht aneinanderliegen (Fig.4). Der Schlitz 30 erstreckt sich in der Ebene, die durch den Mittelpunkt des Katheterschlauches 11 und die Scheitellinie der Trennwand 22 verläuft, so daß die Dicke der Lippen 31, 32 der Stärke der Trennwand 22 entspricht. Bei dem Beispiel der Figuren 1 und 2 ist nur ein Schlitz 30 vorgesehen, der etwa halb so lang wie der Abstand zwischen der Verschmelzung 13 und der ersten spitzennahen Öffnung 25 der Wand 26 ist. Nach Bedarf können mehrere Schlitze 30 in gerader Linie oder auf einer Schraubenlinie parallel zur Längsachse des Katheterschlauches 11 angeordnet sein. Auch bei einem Schlitzverlauf schräg zur Längsachse bleibt die Ventilfunktion des Schlitzes erhalten.

Die Lippen 31, 32 liegen bei geschlossenem Ventil dicht aneinander (Fig.4) und das Hauptlumen 20 ist gegen das Nebenlumen 21 verschlossen. Im Verlauf der Drainage des Körperhohlraumes durch das Hauptlumen 20 entsteht in diesem im Bereich der Spitze 12 Unterdruck, der eine erleichterte Spreizung der Lippen 31, 32 unter dem Einfluß der durch die Öffnung 24 in das Nebenlumen 21 einströmenden gegen die Verschmelzung 13 treffenden Luft zuläßt. Dieser interne Luftzustrom in das Hauptlumen 20 soll durch Druckausgleich ein Ansaugen der Magenschleimhaut gegen die Ränder der Öffnungen 25 in dem Katheterschlauch 11 verhindern. Bei Nachlassen des Luftdruckes in dem Nebenlumen 21 bzw. der Körperflüssigkeitsströmung im Hauptlumen 20 schließt sich der Schlitz 30 unverzüglich und unterbindet durch Verhinderung des Einströmens von Körperflüssigkeit in das Nebenlumen 21 dessen Verengung oder Zusetzung durch Verkrustungen der Körperflüssigkeit.

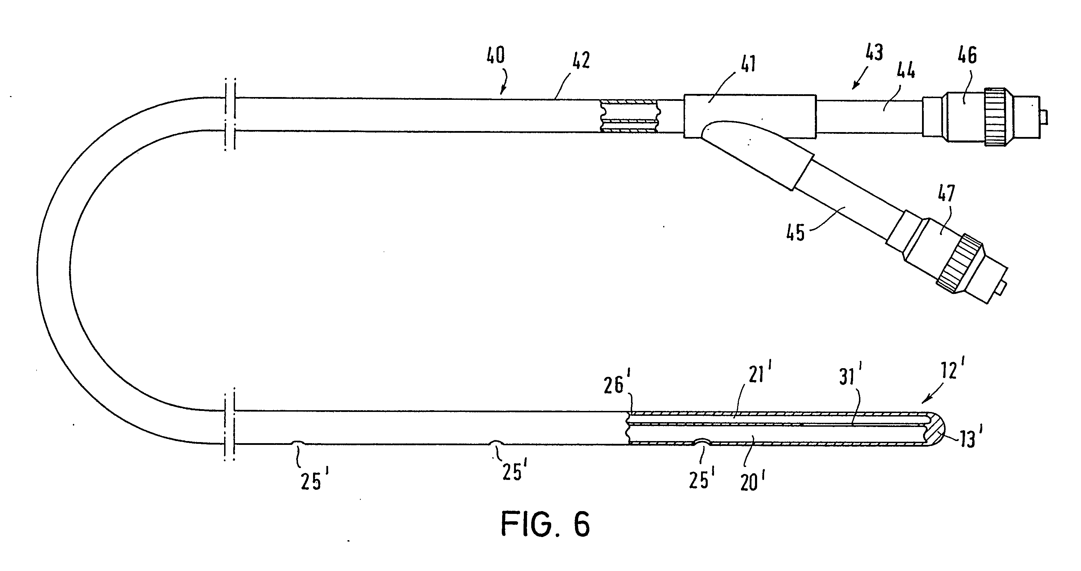

Das Beispiel nach Figur 6 zeigt einen Katheter 40 zur Drainage eines Körperhohlraumes, dessen intrakorporaler Teil den gleichen Aufbau hat wie der Katheter 10 gemäß Figuren 1 bis 5. Entsprechende Teile sind mit gleichen Bezugsziffern unter Hinzufügung von ' gekennzeichnet. Bei dem Katheter 40 dient das Nebenlumen 21' als Spüllumen, durch das Flüssigkeit, ggf. unter Hinzugabe von Medikamenten, die zur Auflösung von Krusten geeignet sind, strömt, um nach Durchtritt durch den Schlitz 30' von der Spitze des Katheters 40 her das Hauptlumen 20' zu spülen und von Ablagerungen freizuhalten. Das patientenferne Ende 41 des Katheterschlauches 42 ist mit einem Y-förmigen Ansatzstück 43 fest verbunden. Das Ansatzstück 43 weist einen zur Längsachse des Katheterschlauches 42 koaxialen Stutzenteil 44 auf, dessen Längskanal mit dem Hauptlumen 20' des Katheterschlauches 42 in Verbindung steht. Ein von dem Ansatzstück 43 schräg abgehender Stutzenteil 45 ist mit seinem Längskanal an das Nebenlumen 21' des Katheterschlauches 42 angeschlossen. Die beiden Stutzenteile 44 und 45 tragen an ihren Enden Anschlußelemente 46, 47, die eine abgedichtete Verbindung mit Anschlußelementen von Systemen zur Zuführung von Spülfluid in das Nebenlumen 21' und zum Abziehen von Körperfluid aus dem Hauptlumen 20' ermöglichen. Bei diesem Katheter 40 entfällt natürlich die Öffnung 24 des Katheters 10 und Luftzutritt in das Nebenlumen 21' wird vermieden.

## Ansprüche

1. Katheter (10;40), bestehend aus einem flexiblen Katheterschlauch (11;42) mit geschlossener Spitze (12;12'), dessen Lumen durch axial verlaufende Trennwände (22;22') in parallele Haupt- und Nebenlumina (20,21; 21,21') unterteilt ist, wobei dem Hauptlumen (20;20') Öffnungen (25;25') in der Wand (26;26') des Katheterschlauches (11;42) zugeordnet sind und mindestens ein Durchlaß in dem spitzennahen Bereich jeder Trennwand (22;22') das Hauptlumen (20;20') mit dem Nebenlumen (21;21') verbindet.

**dadurch gekennzeichnet**, daß der Durchlaß in der Trennwand (22;22') mit gewölbtem Querschnitt als Schlitz (30) ausgebildet ist, dessen Lippen (31,32) dicht aneinanderliegen.

2. Katheter nach Anspruch 1,

**dadurch gekennzeichnet**, daß der Schlitz (30) auf der Scheitellinie der gewölbten Trennwand (22;22') vorgesehen ist.

3. Katheter nach Anspruch 1 oder 2,

**dadurch gekennzeichnet**, daß der Schlitz (30) parallel zur Längsachse des Katheterschlauches (11;42) gerade verläuft.

4. Katheter nach Anspruch 1

**dadurch gekennzeichnet**, daß der Schlitz (30) schräg zur Längsachse des Katheterschlauches (11;42) verläuft.

FIG. 1

FIG. 2

FIG. 3

FIG. 4

FIG. 5

EP 0 321 648 A1

FIG. 6

EP 0 321 648 A1

**Europäisches Patentamt**

## EUROPÄISCHER RECHERCHENBERICHT

Nummer der Anmeldung

## EINSCHLÄGIGE DOKUMENTE

EP 88112684.1

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 3 583 404 (D.McWHORTER)<br>* Gesamt; insbesondere Spalte 2, Zeilen 44-52 *<br>-- | 1 | A 61 M 25/00 |
| A | US - A - 4 676 778 (R.NELSON)<br>* Fig. 1,4-6,8; Spalte 4, Zeilen 38-44,48-50; Spalte 5, Zeilen 16-21,45-52 *<br>-- | 1 | |
| A | US - A - 4 661 110 (E.FORTIER)<br>* Fig. 1,2; Spalte 2, Zeilen 15-33 *<br>---- | 1 | |

|  |  |  | RECHERCHIERTE SACHGEBIETE (Int. Cl.4) |
|---|---|---|---|
|  |  |  | A 61 M 25/00 |

Der vorliegende Recherchenbericht wurde für alle Patentansprüche erstellt.

| Recherchenort | Abschlußdatum der Recherche | Prüfer |
|---|---|---|
| WIEN | 11-04-1989 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82